# EUROPEAN PATENT SPECIFICATION

(11) **EP 4 238 560 B1**
(45) Date of publication and mention of the grant of the patent: **17.09.2025**
(21) Application number: 21886416.3
(22) Date of filing: 29.10.2021
(51) Int. Cl.: A61K 31/27, A61K 31/18, A61P 31/14

(54) **THERAPEUTIC AGENT FOR COVID-19**
THERAPEUTIKUM FÜR COVID-19
AGENT THÉRAPEUTIQUE CONTRE LA COVID-19

(30) Priority: 30.10.2020 JP 2020183229
(43) Date of publication of application: 06.09.2023
(73) Proprietor: Senju Pharmaceutical Co., Ltd., Osaka-shi, Osaka 541-0048 (JP); Tohoku University, Sendai-shi, Miyagi 980-8577 (JP)
(72) Inventor: AZUMA, Mitsuyoshi, Osaka-shi, Osaka 541-0048 (JP); FUKIAGE, Chiho, Osaka-shi, Osaka 541-0048 (JP); IMADA, Takashi, Osaka-shi, Osaka 541-0048 (JP); KISHIMOTO, Yayoi, Osaka-shi, Osaka 541-0048 (JP); HIRASAWA, Noriyasu, Sendai-shi, Miyagi 980-8577 (JP); SAITO, Yoshiro, Sendai-shi, Miyagi 980-8577 (JP); TOYAMA, Takashi, Sendai-shi, Miyagi 980-8577 (JP)
(74) Representative: Mewburn Ellis LLP
(86) International application number: PCT/JP2021/040153
(87) International publication number: WO 2022/092299

(56) References cited:
- WO-A1-2005/056519
- WO-A1-2012/074120
- WO-A1-2017/204166
- WO-A1-2021/175823
- WO-A2-2021/207409
- JP-A- 2006 076 989
- JP-A- H10 147 564
- MA CHUNLONG; SACCO MICHAEL DOMINIC; HURST BRETT; TOWNSEND JULIA ALMA; HU YANMEI; SZETO TOMMY; ZHANG XIUJUN; TARBET BART; MARTY MIC: "Boceprevir, GC-376, and calpain inhibitors II, XII inhibit SARS-CoV-2 viral replication by targeting the viral main protease", CELL RESEARCH, SPRINGER SINGAPORE, SINGAPORE, vol. 30, no. 8, 15 June 2020 (2020-06-15), Singapore , pages 678 - 692, XP037208260, ISSN: 1001-0602, DOI: 10.1038/s41422-020-0356-z
- AMIN SK. ABDUL; BANERJEE SUVANKAR; SINGH SAMAYADITYA; QURESHI INSAF AHMED; GAYEN SHOVANLAL; JHA TARUN: "First structure–activity relationship analysis of SARS-CoV-2 virus main protease (Mpro) inhibitors: an endeavor on COVID-19 drug discovery", MOLECULAR DIVERSITY, SPRINGER INTERNATIONAL PUBLISHING, CHAM, vol. 25, no. 3, 5 January 2021 (2021-01-05), Cham, pages 1827 - 1838, XP037530100, ISSN: 1381-1991, DOI: 10.1007/s11030-020-10166-3
- INOUE JUN, NAKAMURA MASAYUKI, CUI YING-SHE, SAKAI YUSUKE, SAKAI OSAMU, HILL JEANETTE R., WANG KEVIN K. W., YUEN PO-WAI: "Structure-Activity Relationship Study and Drug Profile of N- (4-Fluorophenylsulfonyl)-L-valyl-L-leucinal (SJA6017) as a Potent Calpain Inhibitor.", JOURNAL OF MEDICINAL CHEMISTRY, AMERICAN CHEMICAL SOCIETY, US, vol. 46, no. 5, 1 February 2003 (2003-02-01), US , pages 868 - 871, XP055925927, ISSN: 0022-2623, DOI: 10.1021/jm0201924
- GURARD-LEVIN ZACHARY A.; LIU CHENG; JEKLE ANDREAS; JAISINGHANI RUCHIKA; REN SUPING; VANDYCK KOEN; JOCHMANS DIRK; LEYSSEN PIETER; N: "Evaluation of SARS-CoV-2 3C-like protease inhibitors using self-assembled monolayer desorption ionization mass spectrometry", ANTIVIRAL RESEARCH, ELSEVIER BV, NL, vol. 182, 5 September 2020 (2020-09-05), NL , XP086271090, ISSN: 0166-3542, DOI: 10.1016/j.antiviral.2020.104924
- J ABHITHAJ, FRANCIS DILEEP, C.S. SHARANYA, K.G. ARUN, C. SADASIVAN, VARIYAR E. JAYADEVI: "Repurposing simeprevir, calpain inhibitor IV and a cathepsin F inhibitor against SARS-CoV-2 and insights into their interactions with M pro", JOURNAL OF BIOMOLECULAR STRUCTURE AND DYNAMICS, vol. 40, no. 1, 2 January 2022 (2022-01-02), US , pages 325 - 336, XP009536181, ISSN: 0739-1102, DOI: 10.1080/07391102.2020.1813200
- SCHNEIDER MARTHA, ACKERMANN KERSTIN, STUART MELISSA, WEX CLAUDIA, PROTZER ULRIKE, SCHäTZL HERMANN M., GILCH SABINE: "Severe Acute Respiratory Syndrome Coronavirus Replication Is Severely Impaired by MG132 due to Proteasome-Independent Inhibition of M-Calpain", JOURNAL OF VIROLOGY, THE AMERICAN SOCIETY FOR MICROBIOLOGY, US, vol. 86, no. 18, 15 September 2012 (2012-09-15), US , pages 10112 - 10122, XP055802089, ISSN: 0022-538X, DOI: 10.1128/JVI.01001-12

## Description

### Technical Field

The present disclosure relates to a COVID-19 treatment agent.

### Background Art

Novel coronavirus infection (COVID-19) caused by infection with severe acute respiratory syndrome coronavirus 2 (SARS coronavirus 2: SARS-CoV-2) has recently been raging around the world, and providing a method for treating this disease is an urgent issue. Patent Literature 5 and Non-patent Literature 2 describe some compounds for use in the treatment of this disease.

It is also generally known that viruses mutate to maintain their own survival more efficiently, and there is a need to look at a treatment method for COVID-19 caused by SARS-CoV-2 or mutant strains thereof.

NPL 2 identifies compounds which provide promising starting points for the further development of SARS-CoV-2 therapeutics.

### Citation List

### Patent Literature

| | |
|---|---|
| PTL 1: | JP2006-76989A |
| PTL 2: | WO2012/074120 |
| PTL 3: | WO2017/204166 |
| PTL 4: | JPH10-147564A |
| PTL 5: | WO2021/175823A1 |

### Non-patent Literature

NPL 1: Jun Inoue et al., J. Med. Chem. 2003, 46, 868-871
NPL 2: Ma Chunlong et al., Cell Res. 2020, 30, 678-692

### Summary of Invention

The references to methods of treatment by therapy of this description are to be interpreted as references to compounds, pharmaceutical compositions and medicaments of the present invention for use in those methods.

### Technical Problem

The main object of the present inventors was to develop a method for treating COVID-19.

### Solution to Problem

The present inventors found that a specific compound may be effective in the treatment of COVID-19, and made further improvements.

The present invention provides an agent for use in preventing and/or treating COVID-19, comprising a compound represented by formula (I):

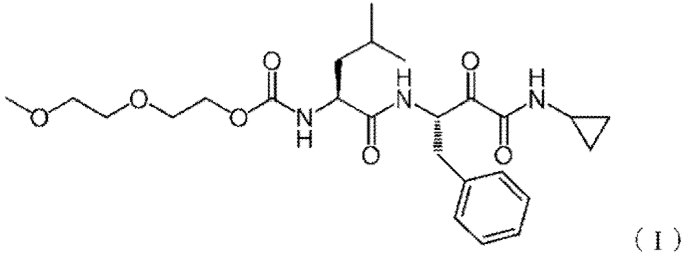

According to an embodiment of the invention, said agent is administered to a person infected with SARS-CoV-2 or a mutant thereof.

Said agent may be administered to an asymptomatic or symptomatic person infected with SARS-CoV-2 or a mutant thereof.

The SARS-CoV-2 mutant may be at least one member selected from the group consisting of α mutant strains, β mutant strains, γ mutant strains, δ mutant strains, λ mutant strains, µ mutant strains, ε mutant strains, η mutant strains, 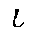 mutant strains, κ mutant strains, ζ mutant strains, and θ mutant strains.

The present disclosure encompasses the following embodiments, which are not of the claimed invention.

An inhibitor for denaturation of cells infected with SARS-CoV-2 or a mutant thereof, comprising at least one compound selected from the group consisting of a compound represented by formula (I): 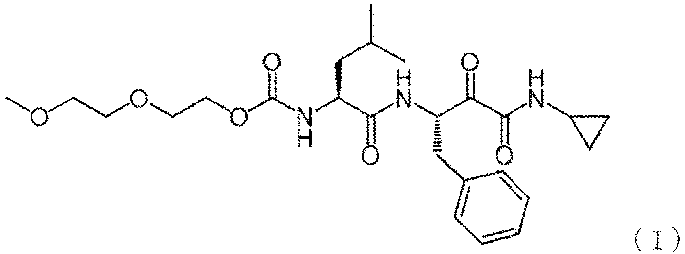 and a compound represented by formula (II): 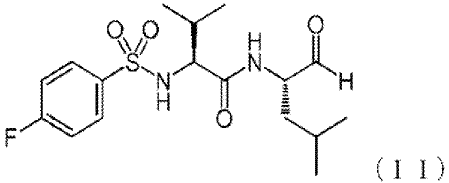 The mutant of SARS-CoV-2 may be at least one member selected from the group consisting of α mutant strains, β mutant strains, γ mutant strains, δ mutant strains, λ mutant strains, µ mutant strains, ε mutant strains, η mutant strains, 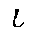 mutant strains, κ mutant strains, ζ mutant strains, and θ mutant strains.

An antiviral drug for inhibiting denaturation of cells infected with SARS-CoV-2 or a mutant thereof in a person infected with SARS-CoV-2 or a mutant thereof, comprising at least one compound selected from the group consisting of a compound represented by formula (I): 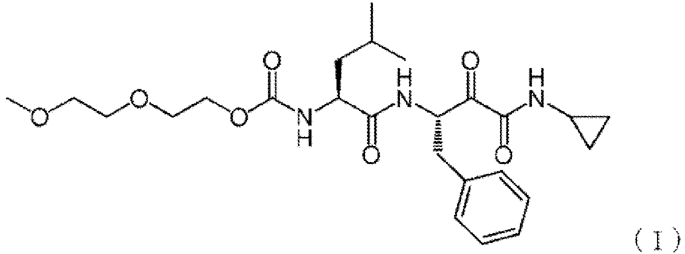 and a compound represented by formula (II): 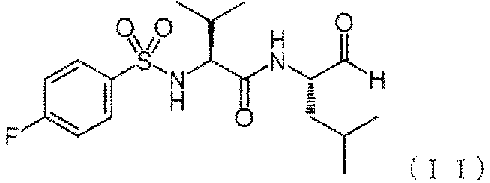 as an active ingredient.

The mutant of SARS-CoV-2 may be at least one member selected from the group consisting of α mutant strains, β mutant strains, γ mutant strains, δ mutant strains, λ mutant strains, µ mutant strains, ε mutant strains, η mutant strains, 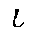 mutant strains, κ mutant strains, ζ mutant strains, and θ mutant strains.

A method for preventing and/or treating COVID-19 caused by SARS-CoV-2 or mutant strains thereof, comprising administering a required amount of at least one compound selected from the group consisting of a compound represented by formula (I): 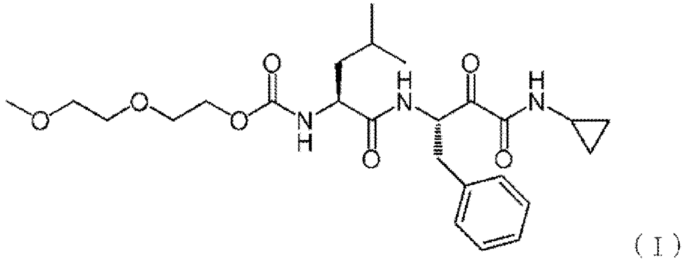 and a compound represented by formula (II): 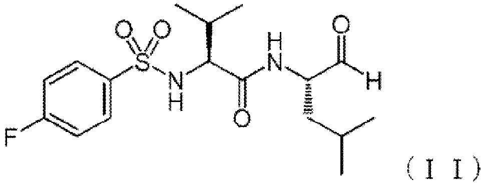 to a subject in need.

The subject in need may be a person infected with SARS-CoV-2 or a mutant thereof.

The subject in need may be an asymptomatic or symptomatic person infected with SARS-CoV-2 or a mutant thereof.

The mutant of SARS-CoV-2 may be at least one member selected from the group consisting of α mutant strains, β mutant strains, γ mutant strains, δ mutant strains, λ mutant strains, µ mutant strains, ε mutant strains, η mutant strains, 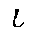 mutant strains, κ mutant strains, ζ mutant strains, and θ mutant strains.

A method for inhibiting denaturation of cells infected with SARS-CoV-2 or a mutant thereof, comprising applying at least one compound selected from a compound represented by formula (I): 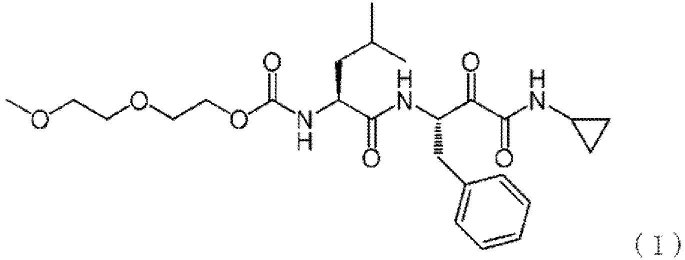 and a compound represented by formula (II): 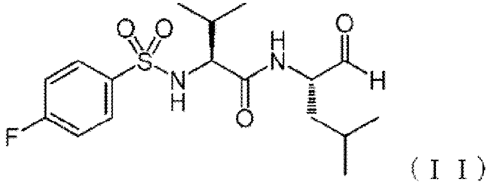 to cells infected with SARS-CoV-2 or a mutant thereof.

The SARS-CoV-2 mutant may at least one member selected from the group consisting of α mutant strains, β mutant strains, γ mutant strains, δ mutant strains, λ mutant strains, µ mutant strains, ε mutant strains, η mutant strains, 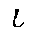 mutant strains, κ mutant strains, ζ mutant strains, and θ mutant strains.

A method for inhibiting denaturation of cells infected with SARS-CoV-2 or a mutant thereof in a person infected with SARS-CoV-2 or a mutant thereof, comprising administering at least one compound selected from the group consisting of a compound represented by formula (I): 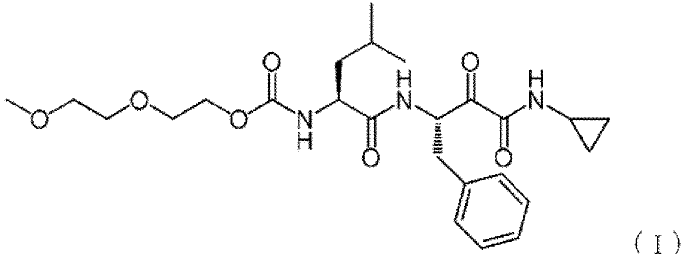 and a compound represented by formula (II): 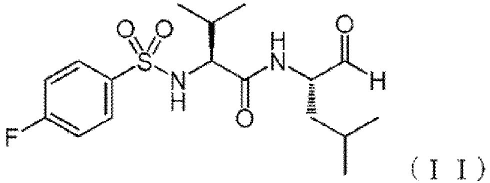 to a person infected with SARS-CoV-2 or a mutant thereof.

The mutant of SARS-CoV-2 may be at least one member selected from the group consisting of α mutant strains, β mutant strains, γ mutant strains, δ mutant strains, λ mutant strains, µ mutant strains, ε mutant strains, η mutant strains, 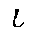 mutant strains, κ mutant strains, ζ mutant strains, and θ mutant strains.

At least one compound for use in prevention and/or treatment of COVID-19, the at least one compound being selected from the group consisting of a compound represented by formula (I): 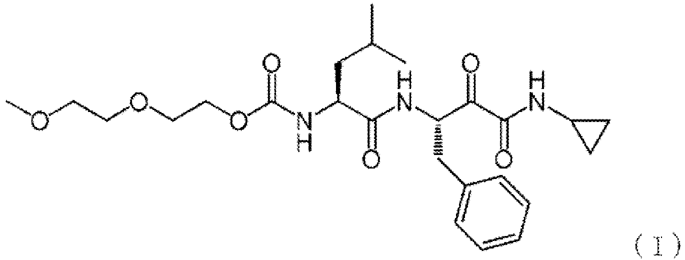 and a compound represented by formula (II):

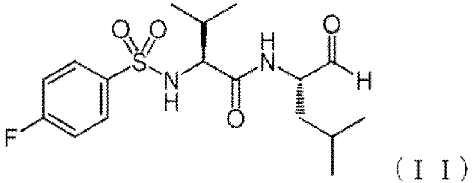

According to an embodiment, said compound is for use in a person infected with SARS-CoV-2 or a mutant thereof.

Said person may be an asymptomatic or symptomatic person infected with SARS-CoV-2 or a mutant thereof.

At least one compound for use in inhibiting denaturation of cells infected with SARS-CoV-2 or a mutant thereof, the at least one compound being selected from the group consisting of a compound represented by formula (I): 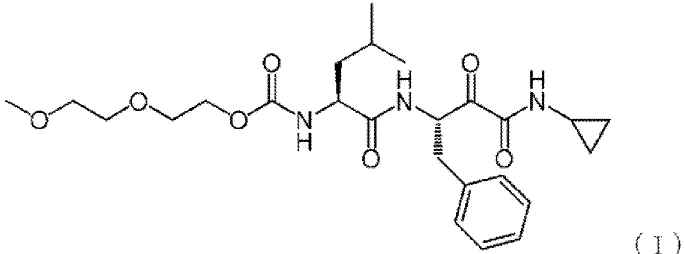 and a compound represented by formula (II):

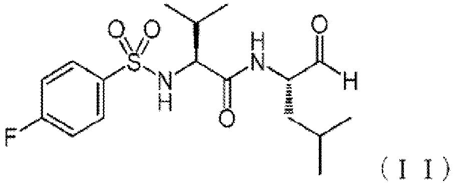

At least one compound for use in inhibiting denaturation of cells infected with SARS-CoV-2 or a mutant thereof in a person infected with SARS-CoV-2 or a mutant thereof, the at least one compound selected from the group consisting of a compound represented by formula (I): 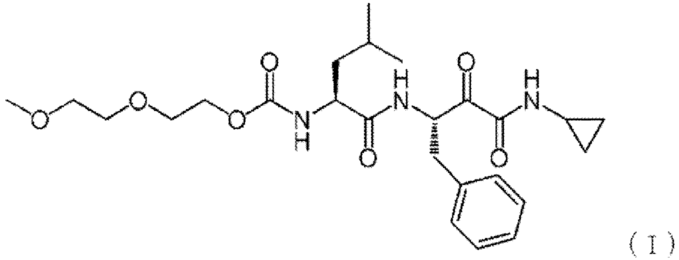 and a compound represented by formula (II):

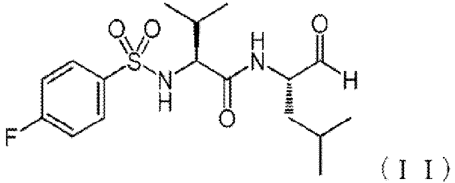

Use of at least one compound for producing a composition (preferably a pharmaceutical composition) for preventing and/or treating COVID-19 caused by SARS-CoV-2 or a mutant strain thereof, the at least one compound being selected from the group consisting of a compound represented by formula (I): 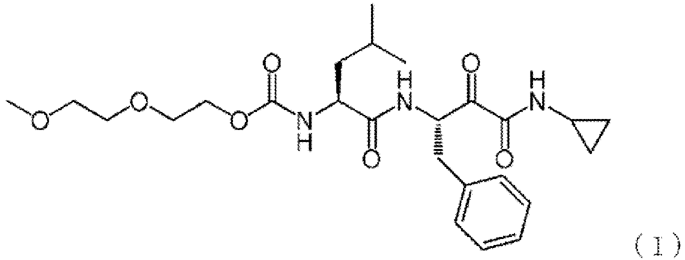 and a compound represented by formula (II):

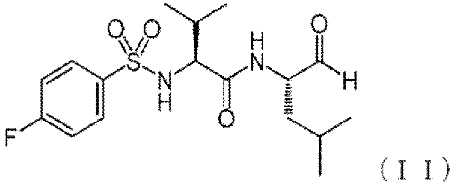

Use of at least one compound for producing a composition (preferably a pharmaceutical composition) for treating a person infected with SARS-CoV-2 or a mutant thereof, the at least one compound being selected from the group consisting of a compound represented by formula (I): 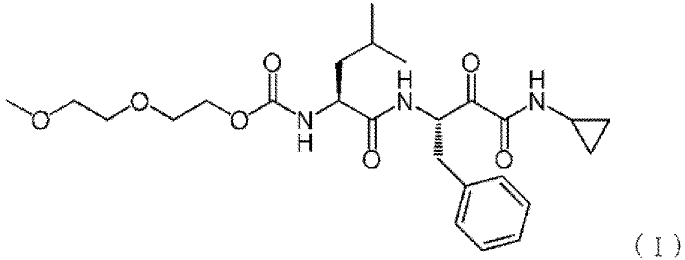 and a compound represented by formula (II):

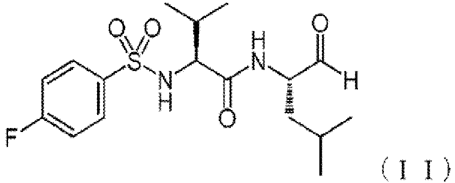

The person infected with SARS-CoV-2 or a mutant thereof may be an asymptomatic or symptomatic person.

Use of at least one compound for producing a composition (preferably a pharmaceutical composition) for inhibiting denaturation of cells infected with SARS-CoV-2 or a mutant thereof, at least one compound being selected from the group consisting of a compound represented by formula (I): 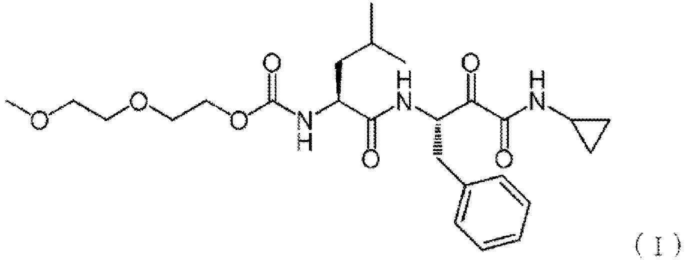 and a compound represented by formula (II):

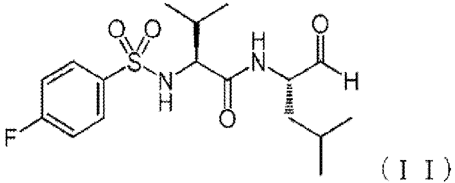

Use of at least one compound for producing a composition (preferably a pharmaceutical composition) for inhibiting denaturation of cells infected with SARS-CoV-2 or a mutant thereof in a person infected with SARS-CoV-2 or a mutant thereof, the at least one compound being selected from the group consisting of a compound represented by formula (I): 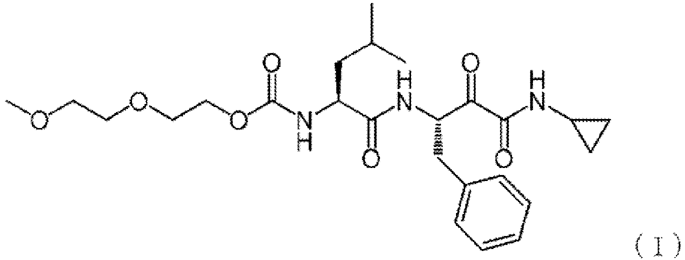 and a compound represented by formula (II):

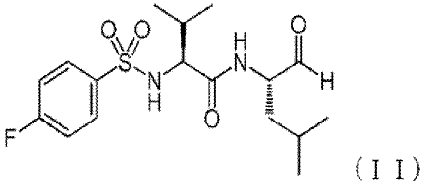

### Advantageous Effects of Invention

The present invention is defined by the appended claims and provides a novel agent for preventing and/or treating COVID-19 caused by SARS-CoV-2 or a mutant strain thereof.

### Brief Description of Drawings

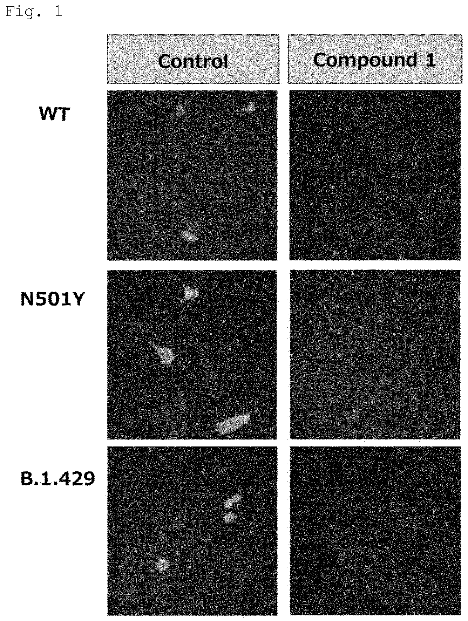

Fig. 1 shows a diagram indicating the effect of exhibiting the compound of the present invention on various mutants described in Examples.

### Description of Embodiments

Embodiments encompassed by the present disclosure are described in more detail below. The present disclosure preferably includes an agent for preventing and/or treating COVID-19 caused by SARS-CoV-2 or a mutant strain thereof; the present invention is defined by the claims. The present disclosure encompasses all that is disclosed in the present specification and that could be acknowledged by a person skilled in the art.

The agent for preventing and/or treating COVID-19 caused by SARS-CoV-2 or a mutant strain thereof encompassed by the present disclosure contains a specific compound that has an effect of inhibiting physiological phenomena caused by SARS-CoV-2 or a mutant strain thereof, and that can act as an active ingredient for COVID-19 caused by SARS-CoV-2 or a mutant strain thereof. The agent for preventing and/or treating COVID-19 caused by SARS-CoV-2 or a mutant strain thereof is sometimes referred to as the treatment agent of the present disclosure.

The compound for use according to the invention is a compound represented by formula (I):

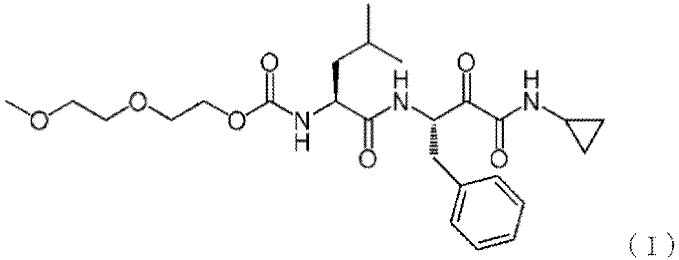

The compound is sometimes referred to as "compound (I)" or "SNJ-1945."

The compound (I) is a known compound and is disclosed in, for example, Patent Literature 1 to 3. The compound (I) can be produced according to the method described in this literature.

The second compound, not of the claimed invention, is a compound represented by formula (II):

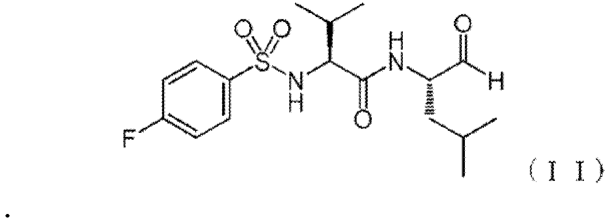

The compound is sometimes referred to as "compound (II)" or "SJA-6017".

The compound (II) is a known compound and is disclosed, for example, in Patent Literature 4 and Non-Patent Literature 1. The compound (II) can be produced according to the method described in this literature.

The compound (I) or compound (II) also includes diastereo mixtures of compound (I) or compound (II) unless otherwise specified.

The treatment agent of the present disclosure may be formed of the compound (I) and/or the compound (II) alone, or may be a composition (particularly a pharmaceutical composition) comprising the compound (I) and/or compound (II) in combination with other components. Pharmaceutically acceptable bases, carriers, additives (e.g., excipients, binders, disintegrants, lubricants, solvents, sweetening agents, coloring agents, taste masking agents, odor-masking agents, surfactants, moisturizing agents, preservatives, pH-adjusting agents, viscosifying agents), etc. are added to the compound (I) and/or compound (II), which are active ingredients, as required. As such bases, carriers, additives, etc., components known in this technical art can be suitably selected and used. The dosage form is not particularly limited. The active ingredient can be mixed with other components in an ordinary method to prepare a preparation in the form of tablets, coated tablets, dispersions, granules, fine granules, capsules, pills, liquids (e.g., injections and infusions), suspensions, emulsions, jelly, chewable agents, or soft tablets.

The amounts of the compound (I) and/or compound (II) in the treatment agent of the present disclosure are not particularly limited, and can be suitably set as long as a treatment effect on COVID-19 is exhibited. The compound is, for example, 0.0005 to 100 mass%, more preferably 0.005 to 90 mass%, and even more preferably 0.05 to 80 mass%.

COVID-19 is an infectious disease characterized by respiratory symptoms caused by a newly specified coronavirus, which is officially named "SARS-CoV-2." It was first reported in Wuhan, Hubei Province, China, in December 2019, and then infection spread widely throughout China and even worldwide. COVID-19 is considered to spread from person to person mainly via airborne droplets dispersed by coughing and sneezing of infected people. Most people infected show no symptoms at all or only mild symptoms; however, some become seriously ill and die. Symptoms include fever, coughing, and shortness of breath. Symptoms usually appear approximately 1 to 14 days after infection.

SARS-CoV-2 is a coronavirus with single-stranded plus-stranded RNA as its genome and is classified in the family *Coronaviridae,* genus β coronavirus, and has a base sequence (SEQ No. 1) shown in NCBI accession number NC_045512. Known examples of coronaviruses that infect humans include four coronaviruses that routinely infect humans (HCoV-229E, HCoV-OC43, HCoV-NL63, and HCoV-HKU1) and two severe pneumonia viruses (SARS-CoV and MERS-CoV) that are transmitted from animals. SARS-CoV-2 is considered to have been transmitted from bats to humans.

Coronaviruses have a bilayer containing lipid as a constituent component, which is referred to as an "envelope" on the outermost side of the particle. Coronaviruses cannot multiply on their own, but can attach to and enter host cells to increase. S protein expressed on the surface of SARS-CoV-2 binds to an ACE2 receptor on the surface of host cells and enables infection of host cells. SARS-CoV-2 is similar to SARS-CoV in that it uses an ACE2 receptor for human infection and differs from MERS-CoV, which uses a DPP-4 receptor. SARS-CoV-2 also differs from other coronaviruses in its structural proteins. Preferable examples of SARS-CoV-2 targeted by the treatment agent of the present disclosure include those having the following homologies. The homology of SARS-CoV-2 and SARS-CoV is about 76% for the S protein, about 90% for the N protein, about 90% for the M protein, and about 94% for the E protein. The homology of SARS-CoV-2 and MERS-CoV is about 35% for the S protein, 48% for the N protein, 42% for the M protein, and 36% for the E protein.

Many reports have reported the emergence of mutant strains in the S protein expressed on the surface of SARS-CoV-2 (Abdul Aleem et al., Emerging Variants of SARS-CoV-2 And Novel Therapeutics Against Coronavirus (COVID-19). StatPearls (Internet): 2021), and the reduced efficacy of the SARS-CoV-2 vaccine is a challenge in the treatment of COVID-19 (Ravindra K Gupta, Nat. Rev. Immunol. 21: 340-341: 2021; Matthew McCallum et al., Science 373: 648-654: 2021).

The treatment agent of the present disclosure exhibits a treatment and/or prevention effect on various types of SARS-CoV-2 mutants, regardless of whether it is pathogenic or not, and is not particularly limited. Examples include a mutant having a base sequence having 70% or more homology to the base sequence shown in SEQ ID No. 1. Examples include a mutant having a base sequence having more preferably 75% or more, even more preferably 80% or more, even more preferably 85% or more, even more preferably 90% or more, and even more preferably 95% or more homology to the base sequence shown in SEQ ID No. 1.

Examples of the mutant include α mutant strains, β mutant strains, γ mutant strains, δ mutant strains, λ mutant strains, µ mutant strains, ε mutant strains, η mutant strains, 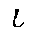 mutant strains, κ mutant strains, ζ mutant strains, and θ mutant strains. These are mutant strains named by the WHO.

Of the SARS-CoV-2 mutants, α mutant strains, β mutant strains, γ mutant strains, ε mutant strains, δ mutant strains, µ mutant strains, or κ mutant strains are preferred. α mutant strains, β mutant strains, γ mutant strains, or ε mutant strains are more preferred.

α mutant strains are mutant strains that were reportedly first discovered in the UK, and are known as strain B.1.1.7. They are mainly known as strains having a mutation such as N501Y.

β mutant strains are mutant strains that were reportedly first discovered in South Africa, and are known as strain B.1.351. They are mainly known as strains having mutations such as N501Y, E484K, and K417N.

γ mutant strains are mutant strains that were reportedly first discovered in Brazil, and are known as strain P.1. They are mainly known as strains having mutations such as N501Y, E484K, and K417T.

δ mutant strains are mutant strains that were reportedly first discovered in India, and are known as strain B.1.617.2. They are mainly known as strains having mutations such as L452R, T478K, D614G, and P681R.

λ mutant strains are mutant strains that were reportedly first discovered in Peru, and are known as strain C.37. They are mainly known as strains having mutations such as L452Q, F490S, D614G, and T859N.

µ mutant strains are mutant strains that were reportedly first discovered in Colombia, and are known as strain B.1.621.1. They are mainly known as strains having mutations such as E484K, N501Y, D614G, and P681H.

ε mutant strains are mutant strains that were reportedly first discovered in the USA, and are known as strain B.1.427/B.1.429. They are mainly known as strains having mutations such as S13L, W152C, L452R, and D614G.

η mutant strains are mutant strains that were reportedly first discovered in the UK and Nigeria, and are known as strain B.1.525. They are mainly known as strains having mutations such as E484K, D614G, and F888L.

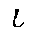 mutant strains are mutant strains that were reportedly first discovered in the USA, and are known as strain B.1.526. They are mainly known as strains having mutations such as E484K, D614G, and A701L.

κ mutant strains are mutant strains that were reportedly first discovered in India, and are known as strain B.1.617.1. They are mainly known as strains having mutations such as L452R, E484Q, D614G, and P681R.

ζ mutant strains are mutant strains that were reportedly first discovered in Brazil, and are known as strain P2 or B,1,1,28.2. They are mainly known as strains having mutations such as E484K, D615G, and V1176F.

θ mutant strains are mutant strains that were reportedly first discovered in the Philippines, and are known as strain P.3 or B.1.1.28.3. They are mainly known as strains having mutations such as E484K, N501Y, D614G, P681H, E1092K, H1101Y, and V1176F.

The subject to whom the treatment agent of the present disclosure is administered is not particularly limited. It is preferably a person infected with SARS-CoV-2 or a mutant thereof, and particularly preferably a subject who has reacted positively to SARS-CoV-2 or a mutant thereof. The positive reaction is performed by a method capable of (preferably specifically) detecting SARS-CoV-2 or a mutant thereof. Examples of the method include a PCR test or an antigen/antibody test (e.g., ELISA) capable of detecting SARS-CoV-2 or a mutant thereof.

The severity of COVID-19 is not particularly limited. The present invention can be used for asymptomatic patients, mildly ill patients, or moderately to severely ill patients. The treatment agent of the present disclosure is effective not only for symptom suppression in those who develop the disease (mild, or moderate to severe), but also for inhibition (reduction) of SARS-CoV-2 or a mutant thereof in the bodies of asymptomatic and symptomatic individuals. Symptoms of the disease are not particularly limited. Examples include fever, dry cough, fatigue, sputum, shortness of breath, sore throat, headache, diarrhea, pneumonia, dyspnoea, etc. Furthermore, the treatment agent of the present invention can be used for the purpose of prevention for subjects not yet infected with SARS-CoV-2 or a mutant thereof.

The target of the treatment agent of the present disclosure includes not only humans but also non-human mammals (in particular non-human mammals infected with SARS-CoV-2 or a mutant thereof). Mammals kept as pets and livestock are particularly preferred as such non-human mammals. Examples include dogs, cats, monkeys, cows, horses, sheep, goats, pigs, rabbits, mice, rats, camels, and llamas. In mammals, as in humans, the treatment agent of the present disclosure can also be used for the purpose of prevention.

The time at which the treatment agent of the present disclosure is administered is not particularly limited, and may be suitably selected, taking into account, for example, the dosage form of preparation, the age of the subject, and the severity of symptoms of the subject. The dosage form is also not particularly limited. For example, oral administration, intravascular (particularly intravenous) administration, and subcutaneous administration are preferable.

The dosage of the treatment agent of the present disclosure may be suitably selected according to the age of the subject, the severity of symptoms of the subject, and other conditions. For example, the amount of the compound (I) in the treatment agent of the present disclosure is preferably 10 to 3000 mg, and more preferably 20 to 2000 mg per adult per day. For example, the amount of the compound (II) in the treatment agent of the present disclosure is preferably 10 to 3000 mg, and more preferably 20 to 2000 mg per adult per day. The treatment agent can be administered once a day or several times a day (preferably two to three times). In the case of mammals, the dosage can be suitably set with reference to the human case. The treatment agent of the present disclosure can be preferably used as a pharmaceutical composition.

As shown in the Examples below, the treatment agent of the present disclosure has an effect of inhibiting the entry of SARS-CoV-2 or a mutant thereof into cells, and an effect of inhibiting the denaturation of infection cells. Accordingly, the treatment agent of the present disclosure is considered to be effective for the prevention of infection by administering it before infection with SARS-CoV-2 or a mutant thereof. For this reason, the treatment agent of the present disclosure can also be preferably used as a preventive agent. In other words, the present disclosure preferably includes a COVID-19 preventive agent and/or a COVID-19 treatment agent. Cellular denaturation refers to physiological and/or morphological changes that occur after infection of cells with a virus. Since the denaturation of SARS-CoV-2 infection cells is considered to be due to the proliferation of SARS-CoV-2, the treatment agent of the present disclosure can be considered to have an effect of inhibiting the proliferation of SARS-CoV-2.

The term "comprising" includes consisting essentially of and consisting of. Further, the present disclosure includes all of any combinations of the constituent requirements described in the present specification.

In addition, the various characteristics (properties, structures, functions, etc.) described in each embodiment of the present disclosure described above may be combined in any way in specifying the main subjects included in the present disclosure. In other words, the present disclosure includes all the main subjects comprising all combinations of the combinable characteristics described in the present specification.

### Examples

The embodiments of the present disclosure are described in more detail below by means of examples; however, the embodiments of the present disclosure are not limited to the Examples below.

### Preparation of compound (examination sample)

As shown in Table 1, compound stock solutions (80 µL of dimethyl sulfoxide (DMSO) solutions containing each compound) were individually placed in a plate, and 40 µL of each stock solution was serially diluted 2 fold to prepare sample solutions. 30 nL of each sample solution was dispensed into a 384-well assay plate by using the ECHO555 acoustic liquid handling system (Labcyte Inc.). Chloroquine, hydroxychloroquine, E64d, and Remdesivir were used as compounds other than SNJ-1945 and SJA-6017. DMSO was dispensed in control wells so that all of the wells had a DMSO concentration of 0.1%.

Chloroquine and hydroxychloroquine are compounds that are considered to have a potential effect on the treatment of COVID-19. Remdesivir is a drug that was approved as a treatment agent of COVID-19 in the US in 2020. It is disclosed that E64d is a protease inhibitor and has antiviral activity by inhibiting the action of proteases of the host. As mentioned above, SNJ-1945 is a compound (I), and SJA-6017 is a compound (II). SJA-6017 is not according to the claimed invention.

**Table 1**

| Compound | Stock solution | Test concentration (µM) 10 points double common ratio | | | | | | | | | |
|---|---|---|---|---|---|---|---|---|---|---|---|
| SNJ-1945 | 30 mM | 30 | 15 | 7.5 | 3.75 | 1.88 | 0.94 | 0.47 | 0.23 | 0.12 | 0.06 |
| SJA-6017 | 30 mM | 30 | 15 | 7.5 | 3.75 | 1.88 | 0.94 | 0.47 | 0.23 | 0.12 | 0.06 |
| Chloroquine | 30 mM | 30 | 15 | 7.5 | 3.75 | 1.88 | 0.94 | 0.47 | 0.23 | 0.12 | 0.06 |
| Hydroxychloroquine | | | | | | | | | | | |
| E64d | | | | | | | | | | | |
| Remdesivir | | | | | | | | | | | |

### Measurement of antiviral effect of compound (CPE assay)

Vero E6 cells (a cell line derived from African green monkey kidney epithelial cells) were cultured in minimum essential medium (MEM) containing 10% fetal bovine serum (FBS), and on the day of assay, the medium was replaced with MEM containing 1% penicillin-streptomycin and 2% FBS. SARS-CoV-2 virus (USA_WA1/2020, M.O.I. to 0.002) was added to Vero E6 cells to achieve a cell viability of 5% after 72 hours of inoculation. An assay plate to which 30 nL of a test compound and 5 µL of medium were added was prepared, and Vero E6 cells after 25 µL of virus inoculation (4000 cells/well) were added thereto, followed by incubation for 72 hours at 37°C, 5% CO₂, and 90% humidity. As a control, Vero E6 cells alone before virus inoculation (100% CPE inhibition group) or Vero E6 cells after virus inoculation alone (0% CPE inhibition group) were set. After 72 hours, Cell-Titer-Glo (Promega Corporation) was added to each well, and incubation for 10 minutes at room temperature was performed. Thereafter, luminescence was measured with a CLARIO star plate reader (BMG Labtech Japan Ltd.).

The cytopathic effect (CPE) is a viral cytopathic effect in host cells, and the Cell-Titer-Glo (Promega Corporation) assay kit is a kit for measuring intracellular ATP as an alternative measurement value of host cell viability. When CPE occurs due to viral infection, the depletion of ATP is measured, which is proportional to viral load; accordingly, the amount of detected ATP is directly proportional to the number of living host cells in culture, allowing the viral cytopathic effect to be quantified.

### Evaluation of cytotoxicity of compound

The cytotoxicity of a compound was evaluated in conjunction with the antiviral effect measurement. An assay plate to which 30 nL of a test compound and 5 µL of medium were added was prepared, and 25 µL of Vero E6 cells (4000 cells/well) were added thereto, followed by incubation for 72 hours at 37°C, 5% CO₂, and 90% humidity. Vero E6 cells alone (survival rate: 100%) and addition of 100 µM Hyamine (survival rate: 0%) were respectively determined to be a high-signal control and a low-signal control. After 72 hours, Cell-Titer-Glo (Promega Corporation) was added to each well, and incubation for 10 minutes at room temperature was performed. Thereafter, luminescence was measured with a PHERAstar plate reader (BMG Labtech Japan Ltd.).

### Data analysis

For all assays, raw data from the plate reader was imported into Activity Base, which is a plate assay data management system.

In the measurement of antiviral effects, signal values were converted into the CPE inhibition rate by using the following formula. CPE inhibition rate (%) = 100 × (each sample value - average value of 0% CPE inhibition group)/(average value of 100% CPE inhibition group - average value of 0% CPE inhibition group)

The survival rate in the cytotoxicity evaluation was calculated using the following formula. Survival rate (%) = 100 × (each sample value - average value of low signal control)/(average value of high signal control - average value of low signal control)

Values of 50% inhibitory concentration (IC₅₀) and 50% cytotoxic concentration (CC₅₀) were calculated from a four-parameter logistic fit of the data by using the Xlfit module of Activity Base.

### Results

The results are shown in Table 2 below. The IC₅₀s of remdesivir, chloroquine, and hydroxychloroquine, whose effects of inhibiting SARS-CoV-2 virus replication were already shown, were 10.56, 2.06, and 6.49 µM, respectively. The IC₅₀ of E64d was 8.23 µM. In contrast, the IC₅₀ of SNJ-1945 and SJA-6017 were 1.52 µM and 0.21 µM, respectively.

The results are summarized in the following table.

**Table 2**

| | CPE reduction (IC50, µM) |
|---|---|
| SNJ-1945 | 1.52 |
| SJA-6017 | 0.21 |
| Chloroquine | 2.06 |
| Hydroxychloroquine | 6.49 |
| E64d | 8.23 |
| Remdesivir | 10.56 |

None of the compounds had cytotoxicity at the concentrations used in the examination.

The results indicate that SNJ-1945 and SJA-6017 have an excellent effect of inhibiting the denaturation of SARS-CoV-2 virus infection cells.

### Effect on various mutants

### 1. Preparation of various corona virus mutants

### Kit marketed by Funakoshi Co., Ltd.

Using pPACK-SPIKE SARS-CoV-2 S Pseudotype Lentivector Packaging Mix (wild type: WT), pPACK-SPIKE N501Y, SARS-CoV-2 S Pseudotype-N501Y Mutant-Lentivector Packaging Mix (N501Y mutant strain), and pPACK-SPIKE B.1.429, and SARS-CoV-2 S Pseudotype-B.1.429 (CAL. 20C) Variant-Lentivector Packaging Mix (B.1.429 mutant strain: ε strain), spike proteins of corona virus mutants of WT, N501Y mutant strain, and S13I/W152C/L452R/D614G(B.1.429) mutant strain (ε strain) were expressed to prepare lentiviruses (sometimes referred to below as "pseudotype viruses") encapsulating GFP expression plasmids, and an experiment to confirm the effect of the compound of the present invention was performed. These pseudotype viruses were concentrated and used for SARS-CoV-2 infection evaluation.

### 2. Evaluation of inhibition of infection (entry) of various coronavirus mutants

ACE2-expressing HEK293T cells (Human ACE2 293T cells, Takara Bio Inc.) were used to evaluate the virus entry inhibition effect of the compound against infection with pseudotype virus particles encapsulating GFP-expressing plasmids. Using SNJ-1945 (compound 1) as a test compound, a DMSO solution of each test compound was added at a concentration of 100 µM, which was the highest concentration at which each test compound did not show cytotoxicity, to a dish in which ACE2-expressing HEK293T cells were seeded, followed by incubation for 30 minutes. Thereafter, the pseudotype viruses (WT, N501Y, and ε mutant strain) were added to examine entry inhibition activity. Infection by these pseudotype virus particles was observed by confocal laser microscopy for fluorescence of GFP, and its inhibitory activity was calculated.

For confocal laser microscopy (Olympus Corporation: FV1000) observation, infection cells were sealed after immobilization with 4% PFA/PBS, and the excitation wavelength and fluorescence wavelength for EGFP were observed. In this experiment, the observation of strong GFP fluorescence intensity means that the pseudotype virus infects ACE2-expressing HEK293T cells. The results are shown in Fig. 1.

The results shown in Fig. 1 reveal that the compound 1 inhibits entry of WT, N501Y mutant strain, and ε mutant strain into cells. N501Y mutation is a mutation found in α, β, and γ mutant strains; accordingly, the compound 1 is expected to inhibit the entry of α, β, and γ mutant strains into cells. Since the ε mutant strain has mutations such as S13L, W152C, L452R, and D614G, the compound 1 is also expected to inhibit the entry of a mutant strain such as ε strain showing a mutation in any of these amino acid sequences, into cells.

### Pharmacokinetic (PK) measurement of SNJ-1945 and SJA-6017

### Test animal

Eight-week-old male S.D. rats were used for the examination of SNJ-1945. Each animal was kept in a room at a temperature of 21.6°C to 22.3°C, a humidity of 47.7% to 60.3%, with lighting for 12 hours/day, and a ventilation frequency of 6 to 20 times/hour. The rats were fasted from the evening of the day before administration, and feeding was resumed after the completion of blood sampling, which was conducted 8 hours after the administration. Water was taken freely.

Male S.D. rats weighing 160 to 210 g were used for the examination of SJA-6017. Each animal was kept in a room at a temperature of 23 ± 2°C, a humidity of 55 ± 10%, with lighting for 12 hours, and a ventilation frequency of 15 times/hour or more. The rats were fasted from the night before administration and feeding was resumed immediately after the administration. Water was taken freely.

### Test method

### 1) Group structure

For the examination of SNJ-1945, four groups of 1, 3, 10, and 30 mg/kg (n=3 for each) were set. For the examination of SJA-6017, two groups of 100 mg/kg (n=3 for each) and 500 mg/kg (n=8 for each) were set.

### 2) Preparation of administration sample

For SNJ-1945, methylcellulose was dissolved in water for injection to a concentration of 1 w/v%. A test substance was suspended in the resultant to prepare samples having concentrations of 0.25 mg/mL (for 1 mg/kg), 0.75 mg/mL (for 3 mg/kg), 2.5 mg/mL (for 10 mg/kg), and 7.5 mg/mL (for 30 mg/kg). The administration solution was dispensed into brown glass bottles.

For SJA-6017, sodium carboxymethylcellulose was dissolved in distilled water to a concentration of 0.5% (w/v). A test substance was suspended in the resultant to prepare samples having concentrations of 10% (for 500 g/kg) and 2% (for 100 mg/kg).

### 3) Administration

SNJ-1945 was orally administered forcibly a single time by using a gastric sonde at doses of 1, 3, 10, and 30 mg/4 mL/kg. SJA-6017 was orally administered forcibly a single time using a gastric sonde at a dose of 5 mL/kg.

### 4) Plasma collection method

In the SNJ-1945 administration group, blood samples were taken over time from the subclavian vein 30 minutes and 1, 2, 4, and 8 hours after administration. The obtained blood was immediately centrifuged at 10000 x g for 3 minutes, and plasma was collected.

In the SJA-6017 administration group, blood samples were collected from the abdominal aorta under ether anesthesia 30 minutes and 1, 2, 4 and 8 hours after administration. The resulting blood was immediately centrifuged at 5,000 rpm for 10 minutes, and plasma was collected.

### 5) Determination of plasma concentration and calculation of pharmacokinetic parameters

### SNJ-1945 administration group

Plasma (20 µL) obtained from the SNJ-1945 administration group was placed in a polypropylene tube, methanol (20 µL) was added thereto, and an internal standard solution (SNJ-1945-d₇; 100 µL) was added thereto, followed by stirring for about 5 minutes. Thereafter, centrifugation (10,000 x g) was performed for 5 minutes, and 100 µL of a supernatant was transferred to another polypropylene tube. 1 mmol/L EDTA-2K (100 µL) was added, followed by stirring for about 10 seconds. The solution was set in an autosampler and measured within 72 hours.

### LC/MS/MS conditions

A Shimadzu 20AD LC System 2 (prominence UFLC_{XR} system; Shimadzu Corporation) and a mass spectrometer (API5000; AB SCIEX) were used as measuring instruments. High-performance liquid chromatography was performed according to the following conditions. Column: CAPCELL CORE AQ, 2.7 µm, 2.1 x 50 mm (Shiseido Japan Co., Ltd.); column temperature: 60°C; mobile phase A: 5 mmol/L ammonium hydrogen carbonate/1 mmol/LEDTA-2K (500:1, v/v); mobile phase B: methanol; flow rate: 0.8 mL/min. Mobile phase gradient conditions were set as shown in Table 3 below.

**Table 3**

| Time (min) | Mobile phase A (%) | Mobile phase B (%) |
|---|---|---|
| 0.0 | 75 | 25 |
| 5.0 | 40 | 60 |
| 5.1 | 75 | 25 |
| 6.0 | 75 | 25 |

| | | |
|---|---|---|
| Linear gradient | | |

The mass spectrometer was set according to the following conditions: ionization mode: turbo ion spray; measurement mode: multiple reaction monitoring; detection mode: negative; collision gas: N₂, 6 (number of valves opened); curtain gas: N₂, 15 psi, ion source gas 1: air, 80 psi; ion source gas 2: air, 80 psi; ion spray voltage: -4500 V; temperature: 500°C; channel electron multiplier: 1800 V or 2000 V. Monitoring ions and dwell time were set as shown in Table 4 below.

**Table 4**

| | Monitoring ions | Dwell time |
|---|---|---|
| SNJ-1945 | m/z 490 → m/z 155 | 50 msec |
| SNJ-1945-d₇ | m/z 497 → m/z 162 | 50 msec |

The measurement results of plasma concentration were analyzed by non-compartmental analysis in the pharmacokinetic analysis software Phoenix WinNonlin Ver. 6.3 (Pharsight Corporation as part of Certara), and each pharmacokinetic parameter was calculated in accordance with the method shown in Table 5 below.

**Table 5**

| | Calculation method |
|---|---|
| Cₘₐₓ | Cₘₐₓ was obtained from the actual measurement value. |
| AUC₀₋₈ | AUC₀₋₈ was calculated by a trapezoidal method using the actual measurement value. C₀ was calculated as 0. |

### Experimental results

Pharmacokinetic parameters (Cₘₐₓ, AUC₀₋₈) at the administration of 1 mg/kg were 186 ng/mL and 167.5 ng·h/mL, pharmacokinetic parameters at the administration of 3 mg/kg were 391 ng/mL and 379.8 ng·h/mL,pharmacokinetic parameters at the administration of 10 mg/kg were 553 ng/mL and 691.1 ng·h/mL,and pharmacokinetic parameters at the administration of 30 mg/kg were 821 ng/mL and 1165.9 ng·h/mL.

### SJA-6017 administration group

Plasma (200 µL) obtained from the SJA-6017 administration group was placed in a brown Spitz tube, and 0.01 M HCl (0.5 mL) was added thereto, and diethyl ether (3 mL) was further added, followed by shaking for 10 minutes. Thereafter, centrifugation (3,000 rpm) for 10 minutes was performed to separate the aqueous layer from the diethyl ether phase. The lower aqueous layer alone was frozen in dry-ice acetone, and the diethyl ether layer was separated into another brown Spitz tube. Thereafter, drying was conducted under reduced pressure, and acetonitrile (100 µL) and a 0.01M hydrochloric acid solution (200 µL) of 5mM phenylhydrazine hydrochloride were added, followed by stirring and dissolving. The solution was transferred to an HPLC vial and allowed to react for 1 hour at 40°C using a water bath. 150 µL of this sample solution was analyzed by HPLC.

### HPLC conditions

Detector: UV absorbance spectrophotometer (measurement
wavelength: 272 nm)
Column: Capcell-pak SG-120 (250 mm)
Column temperature: 40°C
Mobile phase: 0.02 M phosphate buffer (pH of 7.0)/acetonitrile mixture (45:55)
Flow rate: 1.0 mL/min

Cₘₐₓ and AUC₀₋₈ were calculated from the mean plasma concentration at each time point.

### Experimental results

Pharmacokinetic parameters for Cₘₐₓ and AUC₀₋₈ at the administration of 100 mg/kg were respectively 284 ng/mL and 678.9 ng·h/mL,and pharmacokinetic parameters for Cₘₐₓ and AUC₀₋₈ at the administration of 500 mg/kg were respectively 348 ng/mL and 1837.3 ng·h/mL.

The results were summarized and shown in Table 6.

**Table 6**

| | Dose (mg/kg) | Cₘₐₓ (ng/mL) | AUC₀₋₈ (ng·h/mL) |
|---|---|---|---|
| SNJ-1945 | 1 | 186 | 167.5 |
| | 3 | 391 | 379.8 |
| | 10 | 553 | 691.1 |
| | 30 | 821 | 1165.9 |
| SJA-6017 | 100 | 284 | 678.9 |
| | 500 | 348 | 1837.3 |

The low dose (30 mg/kg) of SNJ-1945 showed a high blood concentration as compared to the high dose (100 mg/kg) of SJA-6017. Assuming a dose-proportional increase in blood concentration, SNJ-1945 showed about a five to ten times higher blood concentration difference.

The results indicate that SJA-6017 has stronger activity of inhibiting denaturation of SARS-CoV-2 infection cells than that of SNJ-1945 (IC₅₀ being as follows: SNJ-1945: 1.52 µM and SJA-6017: 0.21 µM). However, since the blood concentration of SNJ-1945 is about 5 to 10 times higher, it is predicted that SNJ-1945 has a higher distribution to target tissues and that SNJ-1945 has a stronger SARS-CoV-2 inhibition activity in tissues than that of SJA-6017.

## Claims

1. An agent for use in preventing and/or treating COVID-19, comprising a compound represented by formula (I):

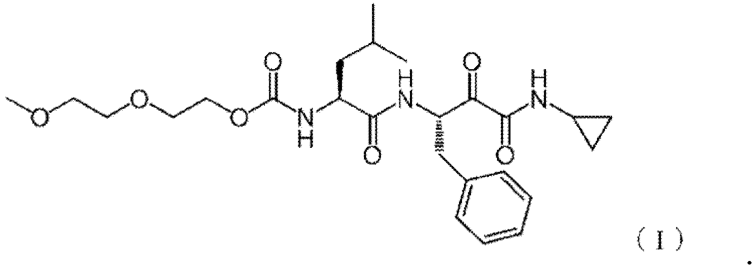

2. The agent for use according to claim 1, which is administered to a person infected with SARS-CoV-2 or a mutant thereof.

3. The agent for use according to claim 2, which is administered to an asymptomatic or symptomatic person infected with SARS-CoV-2 or a mutant thereof.

4. The agent for use according to claim 2 or 3, wherein the SARS-CoV-2 mutant is at least one member selected from the group consisting of α mutant strains, β mutant strains, γ mutant strains, δ mutant strains, λ mutant strains, µ mutant strains, ε mutant strains, η mutant strains, 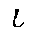 mutant strains, κ mutant strains, ζ mutant strains, and θ mutant strains.

## Patentansprüche

1. Mittel zur Verwendung bei der Prävention und/oder Behandlung von COVID-19, das eine Verbindung umfasst, die durch die Formel (I) dargestellt ist:

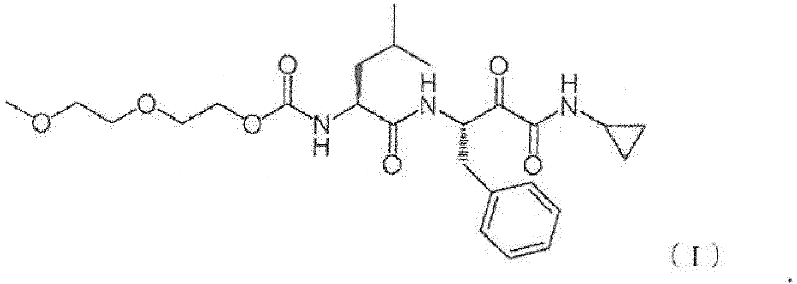

2. Mittel zur Verwendung nach Anspruch 1, das einer Person verabreicht wird, die mit SARS-CoV-2 oder einer Mutante davon infiziert ist.

3. Mittel zur Verwendung nach Anspruch 2, das einer asymptomatischen oder symptomatischen Person verabreicht wird, die mit SARS-CoV-2 oder einer Mutante davon infiziert ist.

4. Mittel zur Verwendung nach Anspruch 2 oder 3, wobei die SARS-CoV-2-Mutante zumindest ein aus der aus α-Mutantenstämmen, β-Mutantenstämmen, γ-Mutantenstämmen, δ-Mutantenstämmen, λ-Mutantenstämmen, µ-Mutantenstämmen, ε-Mutantenstämmen, n-Mutantenstämmen, 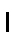 -Mutantenstämmen, κ-Mutantenstämmen, ζ-Mutantenstämmen und θ-Mutantenstämmen bestehenden Gruppe ausgewähltes Mitglied ist.

## Revendications

1. Agent pour utilisation dans la prévention et/ou le traitement de la COVID-19, comprenant un composé représenté par la formule (I) :

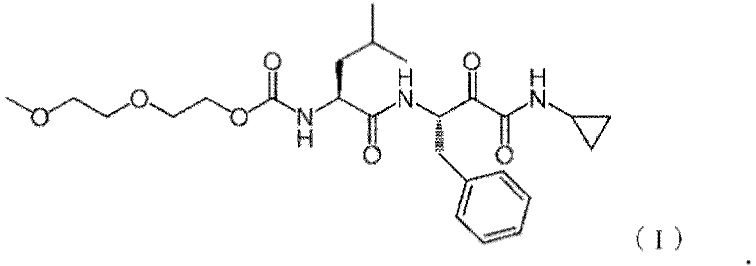

2. Agent pour utilisation selon la revendication 1, qui est administré à une personne infectée par le SARS-CoV-2 ou un mutant de celui-ci.

3. Agent pour utilisation selon la revendication 2, qui est administré à une personne asymptomatique ou symptomatique infectée par le SARS-CoV-2 ou un mutant de celui-ci.

4. Agent pour utilisation selon la revendication 2 ou 3, dans lequel le mutant de SARS-CoV-2 est au moins un élément choisi dans le groupe constitué de souches mutantes α, de souches mutantes β, de souches mutantes γ, de souches mutantes δ, de souches mutantes λ, de souches mutantes µ, de souches mutantes ε, de souches mutantes η, de souches mutantes 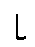 , de souches mutantes K, de souches mutantes ζ et de souches mutantes θ.
